# EUROPEAN PATENT APPLICATION

(11) **EP 4 803 026 A1**
(43) Date of publication of application: **09.09.2026**
(21) Application number: 26161982.9
(22) Date of filing: 03.03.2026
(51) Int. Cl.: A61B 34/10, A61B 34/20

(54) **MEDICAL DEVICE AND OPERATION METHOD THEREOF**

(30) Priority: 04.03.2025 JP 2025033503
(71) Applicant: FUJIFILM Corporation, Tokyo 106-8620 (JP)
(72) Inventor: MACHII, Yusuke, Tokyo, 106-8620 (JP)
(74) Representative: Dehns Germany Partnerschaft mbB

(57) **Abstract**

The present disclosure provides a medical device (14) and an operation method thereof that can present posture information of a 3D organ model (32) that a surgeon wants to see during surgery.

A 3D organ model acquisition unit (20) acquires a 3D organ model (32) corresponding to an observation target organ (30a). An observation video acquisition unit (21) acquires an observation video (30) of an observation target including the observation target organ (30a). A scene information estimation unit (22) estimates scene information determined based on a position of the observation target organ (30a) or a positional relationship between the observation target organ (30a) and a member used for the observation target organ (30a), from the observation video (30). A scene-specific posture information estimation unit (23) estimates posture information of the 3D organ model (32) based on the scene information as scene-specific posture information. A display controller (24) controls the display of the 3D organ model (32) based on the scene-specific posture information.

## Description

### BACKGROUND OF THE INVENTION

### 1. Field of the Invention

The present disclosure relates to a medical device and an operation method thereof.

### 2. Description of the Related Art

JP2015-83040A (corresponding to US2016/0228075A1) discloses generating an observation image in which an inside of a subject is visualized with a distal end position of a virtual medical instrument as a viewpoint, and determining whether the distal end position is within an unallowable distance of a predetermined anatomical structure (organ).

### SUMMARY OF THE INVENTION

In a case of resecting an organ, it is important to understand where to start cutting the organ and which blood vessel is visible during the resection in order to perform the surgery safely. Currently, a surgeon often makes determinations mentally by associating a structure in an intraoperative laparoscopic video with a structure in a preoperative 3D organ model. Therefore, it is required to present posture information of a 3D organ model that the surgeon wants to see during the surgery.

An object of the present disclosure is to provide a medical device and an operation method thereof that can present posture information of a 3D organ model that a surgeon wants to see during surgery.

According to the present disclosure, there is provided a medical device comprising: a processor, in which the processor acquires a 3D organ model corresponding to an observation target organ, acquires an observation video of an observation target including the observation target organ, estimates scene information determined based on a position of the observation target organ or a positional relationship between the observation target organ and a member used for the observation target organ, from the observation video, estimates posture information of the 3D organ model based on the scene information as scene-specific posture information, and controls display of the 3D organ model based on the scene-specific posture information.

It is preferable that the member be a surgical tool used for resection of the observation target organ, and the processor estimate first scene information according to a relative posture between a camera that captures the observation video and the observation target organ in a case where the surgical tool is not included in the observation video, and estimate first scene-specific posture information as the posture information based on the first scene information, and estimate at least one of second scene information or third scene information according to either a position of the surgical tool and a position of the observation target organ, or a surgical tool-organ distance between the surgical tool and the observation target organ in a case where the surgical tool is included in the observation video, and estimate at least one of second scene-specific posture information as the posture information based on the second scene information or third scene-specific posture information as the posture information based on the third scene information. It is preferable that the second scene information be estimated in a case where the surgical tool-organ distance exceeds a first threshold value, and the third scene information be estimated in a case where the surgical tool-organ distance is equal to or less than the first threshold value.

It is preferable that, in a case where the processor performs any of control of the display based on the first scene-specific posture information, control of the display based on the second scene-specific posture information, or control of the display based on the third scene-specific posture information, any of first display of displaying the 3D organ model that changes in conjunction with a posture of the observation target organ, second display of displaying the 3D organ model as viewed from a line of sight connecting a distal end part of the surgical tool and a center of a planned resection surface of the observation target organ, third display of displaying the 3D organ model as viewed from a direction of the planned resection surface of the observation target organ, fourth display of displaying the 3D organ model as viewed from a line of sight connecting the distal end part of the surgical tool and a blood vessel of interest included in the observation target organ, or fifth display of displaying the 3D organ model as viewed from a line of sight connecting the distal end part of the surgical tool and a center or center of gravity of the observation target organ be performed.

It is preferable that the member be an ultrasound probe, and the processor estimate first scene information according to a relative posture between a camera that captures the observation video and the observation target organ in a case where the ultrasound probe is not included in the observation video, and estimate first scene-specific posture information as the posture information based on the first scene information, and estimate at least one of fourth scene information or fifth scene information according to either a position of the ultrasound probe and a position of the observation target organ, or an organ-probe distance between the ultrasound probe and the observation target organ in a case where the ultrasound probe is included in the observation video, and estimate at least one of fourth scene-specific posture information as the posture information based on the fourth scene information or fifth scene-specific posture information as the posture information based on the fifth scene information. It is preferable that the fourth scene information be estimated in a case where the organ-probe distance exceeds a second threshold value, and the fifth scene information be estimated in a case where the organ-probe distance is equal to or less than the second threshold value.

It is preferable that, in a case where the processor performs any of control of the display based on the first scene-specific posture information, control of the display based on the fourth scene-specific posture information, or control of the display based on the fifth scene-specific posture information, any of first display of displaying the 3D organ model that changes in conjunction with a posture of the observation target organ, sixth display of displaying the 3D organ model as viewed from a direction intersecting a surface intersecting an element surface of the ultrasound probe, or seventh display of displaying the 3D organ model as viewed from a direction intersecting the element surface of the ultrasound probe be performed.

It is preferable that the processor determine whether or not the ultrasound probe is in contact with the observation target organ based on an ultrasound video obtained from the ultrasound probe, and estimate any of the fourth scene information or the fifth scene information according to any of a result of the determination or a combination of the result of the determination and the observation video. It is preferable that the medical device further comprise a camera that captures the observation video, and the processor determine movement of the camera, and estimate the scene-specific posture information in a case where it is determined that the camera has moved.

According to the present disclosure, there is provided an operation method of a medical device including a processor, the operation method comprising causing the processor to execute: a step of acquiring a 3D organ model corresponding to an observation target organ; a step of acquiring an observation video of an observation target including the observation target organ; a step of estimating scene information determined based on a position of the observation target organ or a positional relationship between the observation target organ and a member, from the observation video; a step of estimating posture information of the 3D organ model based on the scene information as scene-specific posture information; and a step of controlling display of the 3D organ model based on the scene-specific posture information.

According to the present disclosure, it is possible to present posture information of a 3D organ model that the surgeon wants to see during the surgery.

### BRIEF DESCRIPTION OF THE DRAWINGS

FIG. 1 is a schematic diagram of a medical system.
FIG. 2 is a block diagram showing functions of a medical image processing device.
FIGS. 3A and 3B are image diagrams of a display that displays a laparoscopic video and a 3D organ model.
FIG. 4 is a block diagram showing functions of a scene information estimation unit and a scene-specific posture information estimation unit that estimate first scene information to third scene information.
FIG. 5A is an explanatory diagram showing a surgical tool-organ distance exceeding a first threshold value, and FIG. 5B is an explanatory diagram showing a surgical tool-organ distance equal to or less than the first threshold value.
FIG. 6 is an explanatory diagram showing a 3D organ model that changes in conjunction with an observation target organ.
FIG. 7 is an explanatory diagram showing the 3D organ model as viewed from a line of sight VL1.
FIG. 8 is an explanatory diagram showing the 3D organ model as viewed from a direction DA.
FIG. 9 is an explanatory diagram showing the 3D organ model as viewed from a line of sight VL2.
FIG. 10 is an explanatory diagram showing the 3D organ model as viewed from a line of sight VL3.
FIG. 11 is a block diagram showing functions of a scene information estimation unit and a scene-specific posture information estimation unit that estimate first scene information, fourth scene information, and fifth scene information.
FIG. 12A is an explanatory diagram showing an organ-probe distance exceeding a second threshold value, and FIG. 12B is an explanatory diagram showing an organ-probe distance equal to or less than the second threshold value.
FIG. 13 is an explanatory diagram showing the 3D organ model as viewed from a direction DB.
FIG. 14 is an explanatory diagram showing the 3D organ model as viewed from a direction DC.
FIG. 15A is a block diagram showing functions of a contact determination unit and a scene information estimation unit that use a determination result, and FIG. 15B is a block diagram showing functions of a contact determination unit and a scene information estimation unit that use the determination result and a laparoscopic video.
FIG. 16 is a block diagram showing functions of a camera movement determination unit and a scene information estimation unit.
FIG. 17 is a flowchart showing a series of flows of the present disclosure.

### DESCRIPTION OF THE PREFERRED EMBODIMENTS

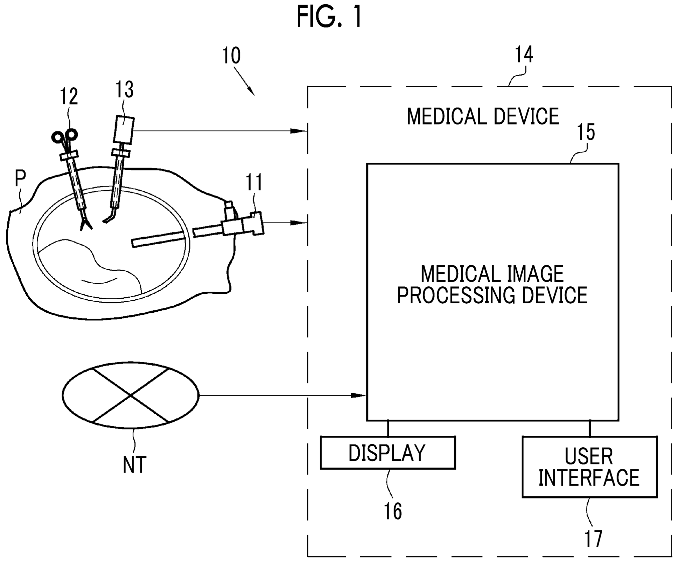

As shown in FIG. 1, a medical system 10 comprises a laparoscope 11, a surgical tool 12, an ultrasound probe 13, and a medical device 14. The laparoscope 11 captures an image of an inside of a body of a patient P and transmits a laparoscopic video obtained by the capturing to the medical device 14. The surgical tool 12 is used for resection of an observation target organ. The ultrasound probe 13 acquires an ultrasound video of an observation target and transmits the ultrasound video to the medical device 14. The laparoscope 11 is also connected to a light source device (not shown), and illumination light from the light source device is supplied to the laparoscope 11.

The medical device 14 comprises a medical image processing device 15 configured by a computer such as a server, a display 16, and a user interface 17. In addition, the medical image processing device 15 is connected to a network NT. A picture archiving and communication system (PACS) or the like is connected to the network NT, and various image data and the like from the PACS are incorporated into the medical image processing device 15 via the network NT.

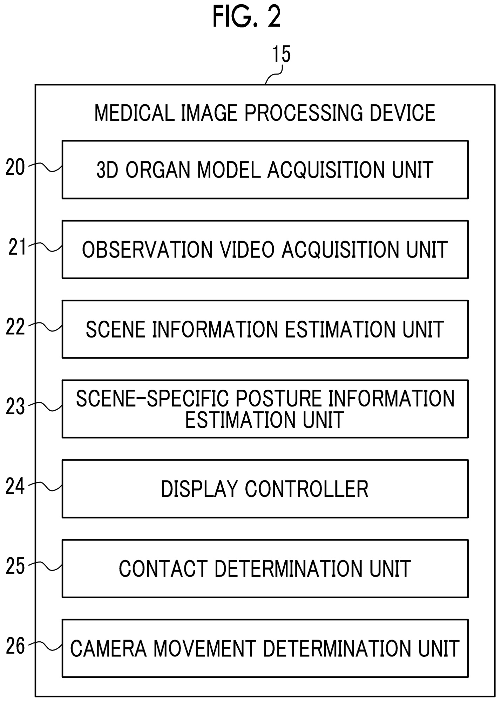

In the medical image processing device 15, a program for executing various types of processing is stored in a program memory (not shown). A central controller (not shown) configured by a processor executes the program in the program memory, whereby the medical image processing device 15 implements functions of a 3D organ model acquisition unit 20, an observation video acquisition unit 21, a scene information estimation unit 22, a scene-specific posture information estimation unit 23, a display controller 24, a contact determination unit 25, and a camera movement determination unit 26 as shown in FIG. 2.

The 3D organ model acquisition unit 20 acquires a 3D organ model corresponding to the observation target organ. The 3D organ model is acquired from a 3D organ model image server (not shown) or the like via the network NT. The 3D organ model is a model extracted from a radiation image such as an X-ray image or a CT image, or an MRI image.

The observation video acquisition unit 21 acquires an observation video of an observation target including the observation target organ. In the present embodiment, the laparoscopic video obtained by the laparoscope 11 is acquired as the observation video. The term "observation" refers to a period in which a user observes a video for observation such as the laparoscopic video, and includes not only a period in which the observation target organ is observed but also a period in which various surgeries such as resection of the observation target organ using the surgical tool 12, which is one of members, are performed and a period in which the observation target organ is diagnosed using the ultrasound probe 13, which is one of members, during the observation.

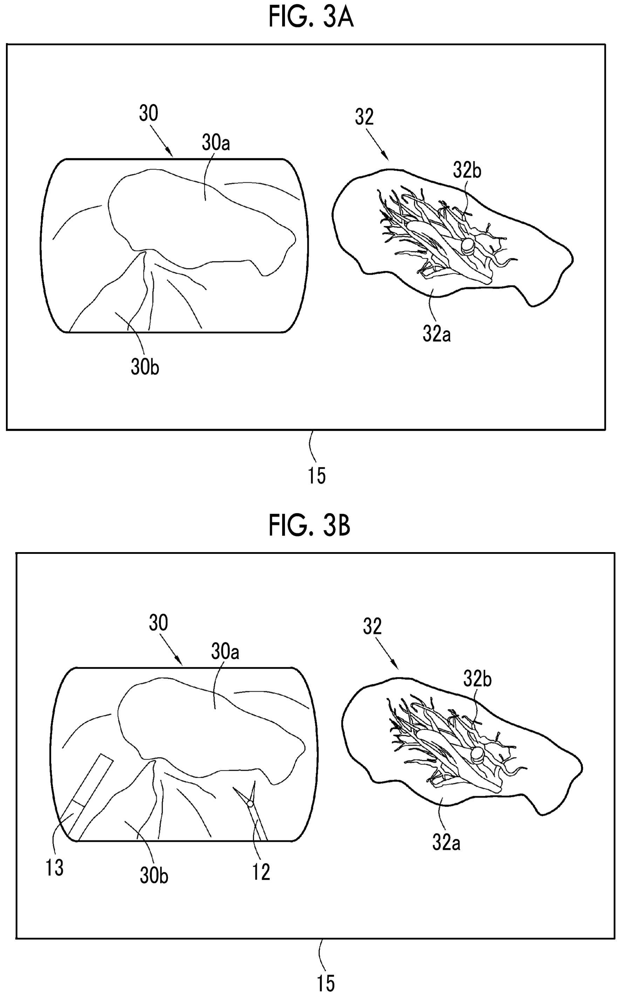

The observation image may include or may not include the member used for the observation target organ. Specifically, in a case where the member used for the observation target is not included, as shown in FIG. 3A, a laparoscopic video 30 may include not only a liver 30a but also a structure 30b around the liver 30a. On the other hand, in a case where the member used for the observation target organ is included, as shown in FIG. 3B, the laparoscopic video 30 may include the liver 30a, the structure 30b around the liver 30a, the surgical tool 12, the ultrasound probe 13, and the like.

The observation target organ currently being observed corresponds to the liver 30a, and the observation target includes the liver 30a, as well as the structure 30b around the liver 30a, the surgical tool 12, and the ultrasound probe 13. The laparoscopic video 30 is displayed on the display 16 together with a 3D organ model 32. The 3D organ model 32 displays an internal blood vessel 32b of the liver on a liver 32a. The display of the laparoscopic video 30 and the 3D organ model 32 is controlled by the display controller 24. In addition to the liver, the observation target organ may be, for example, a kidney, a pancreas, a spleen, a uterus, a lung, a bronchus, an intracranial blood vessel, a prostate, or a nerve, and is not limited to the above organs and may be various other organs.

It is preferable that the observation video be a color video, and various medical videos other than the laparoscopic video may be used. For example, the observation image may be an ultrasound video obtained by the ultrasound probe 13. In addition, in a case of the laparoscopic video, the video may be a monocular video captured by a single imaging sensor, or stereo videos captured by a plurality of imaging sensors.

The scene information estimation unit 22 estimates scene information determined based on a position of the observation target organ or a positional relationship between the observation target organ and a member used for the observation target organ, from the laparoscopic video. It is preferable that the scene information be capable of discriminating or separating situations during the observation. The scene-specific posture information estimation unit 23 estimates posture information of the 3D organ model based on the scene information as scene-specific posture information. The scene-specific posture information is assumed to be expressed in a three-dimensional coordinate system (Viewer system) of XYZ axes, but is not limited to this. The display controller 24 controls the display of the 3D organ model based on the scene-specific posture information. The scene-specific posture information estimation unit 23 may estimate the posture information of the 3D organ model as virtual viewpoint information or line-of-sight information instead of the scene-specific posture information.

The scene information is assumed to include the presence or absence of the surgical tool 12, the discrimination of the surgical tool 12, the position and/or posture of the surgical tool 12, the position and/or posture of the observation target organ, the presence or absence of the resection, the presence or absence of the adverse event, and the like, but is not particularly limited. In addition, the scene information may be obtained by dividing the scene into categories taking into consideration of treatment target information (for example, sub-region information) or surgical procedure information (partial resection, subsegmental resection, or wedge resection), or by combining these. In addition, it is preferable that the scene information estimation unit 22 be a learning model that has been trained using the laparoscopic video and the scene information. In addition, it is preferable that the scene-specific posture information estimation unit 23 be a learning model that has been trained using the scene information, or the scene information, the laparoscopic video, and the posture information of the 3D organ model.

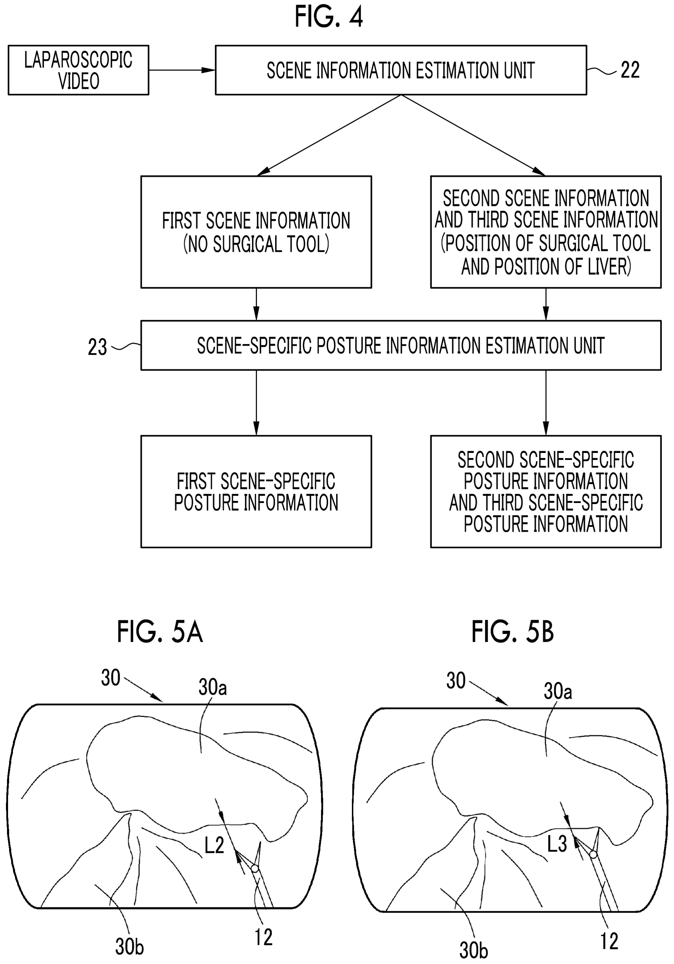

Specifically, as shown in FIG. 4, in a case where the member is the surgical tool 12 used for resection of the observation target organ, the scene information estimation unit 22 estimates first scene information according to a relative posture between the laparoscope 11, which serves as a camera that captures the laparoscopic video, and the observation target organ in a case where the surgical tool is not included in the observation video. Then, the scene-specific posture information estimation unit 23 estimates first scene-specific posture information as the posture information based on the first scene information.

On the other hand, in a case where the surgical tool is included in the observation video, at least one of second scene information or third scene information is estimated according to the position of the surgical tool and the position of the observation target organ. Then, the scene-specific posture information estimation unit 23 estimates at least one of second scene-specific posture information as the posture information based on the second scene information or third scene-specific posture information as the posture information based on the third scene information. As shown in FIG. 5A, it is preferable that the second scene information be estimated in a case where a surgical tool-organ distance L2 exceeds a first threshold value. In addition, as shown in FIG. 5B, it is preferable that the third scene information be estimated in a case where a surgical tool-organ distance L3 is equal to or less than the first threshold value. It is preferable that the surgical tool-organ distance be a distance between a distal end of the surgical tool 12 and the observation target organ (liver 30a).

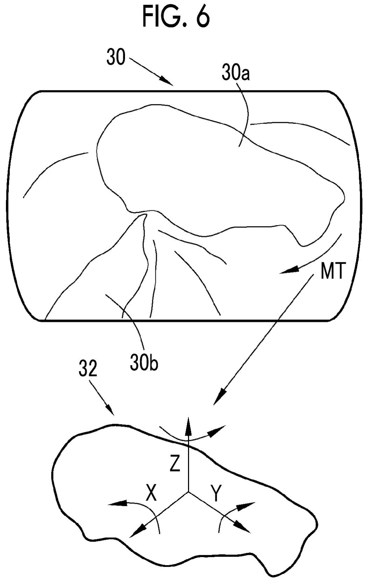

It is preferable that, in a case where the display controller 24 performs any of control of display based on the first scene-specific posture information, control of the display based on the second scene-specific posture information, or control of the display based on the third scene-specific posture information, any of first display to fifth display be performed as display of a 3D organ model. In the first display, as shown in FIG. 6, a 3D organ model that changes in conjunction with the posture of the liver 30a is displayed. The display of the 3D organ model changes in accordance with movement MT of the liver 30a. The display controller 24 controls the display by rotating the 3D organ model in each of three axial directions of the X-axis, the Y-axis, and the Z-axis based on the line of sight. The center in the present embodiment includes not only the center but also the vicinity of the center.

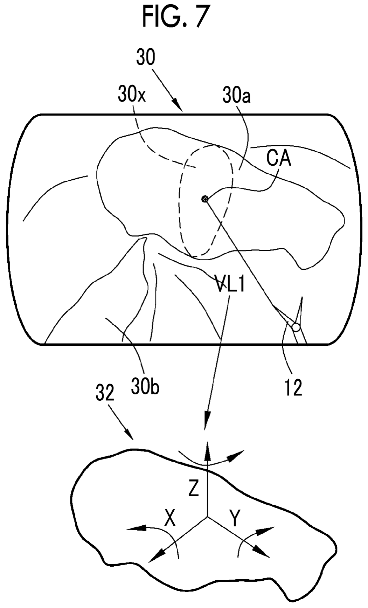

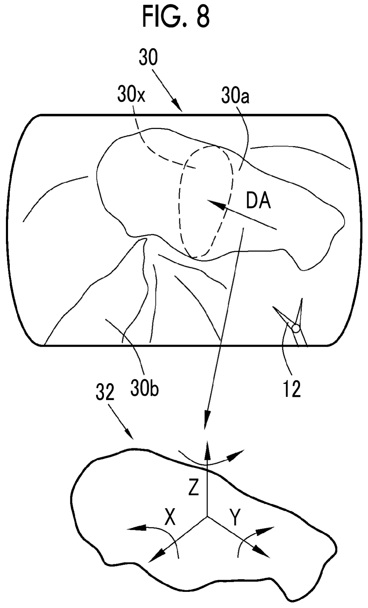

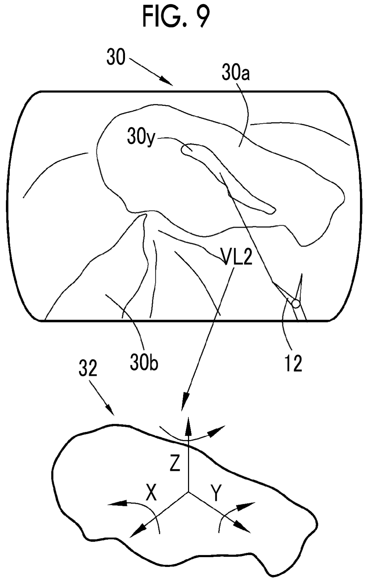

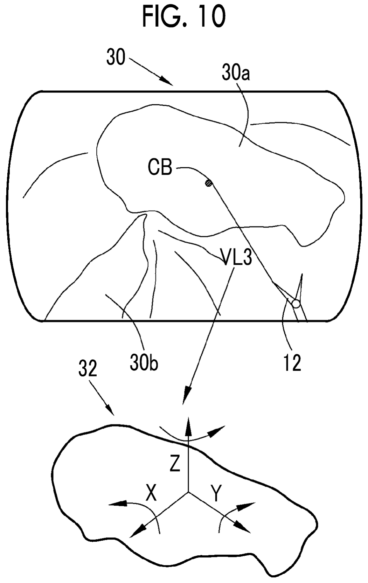

In the second display, as shown in FIG. 7, a 3D organ model as viewed from a line of sight VL1 connecting a distal end part of the surgical tool 12 and a center CA of a planned resection surface 30x (or a resection cross section (surface actually resected)) of the liver 30a is displayed. In the third display, as shown in FIG. 8, a 3D organ model as viewed from a direction DA of the planned resection surface 30x of the liver 30a is displayed. In the fourth display, as shown in FIG. 9, a 3D organ model as viewed from a line of sight VL2 connecting the distal end part of the surgical tool 12 and a blood vessel of interest 30y included in the liver 30a is displayed. In the fifth display, as shown in FIG. 10, a 3D organ model as viewed from a line of sight VL3 connecting the distal end part of the surgical tool 12 and the center CB (or center of gravity) of the liver 30a is displayed.

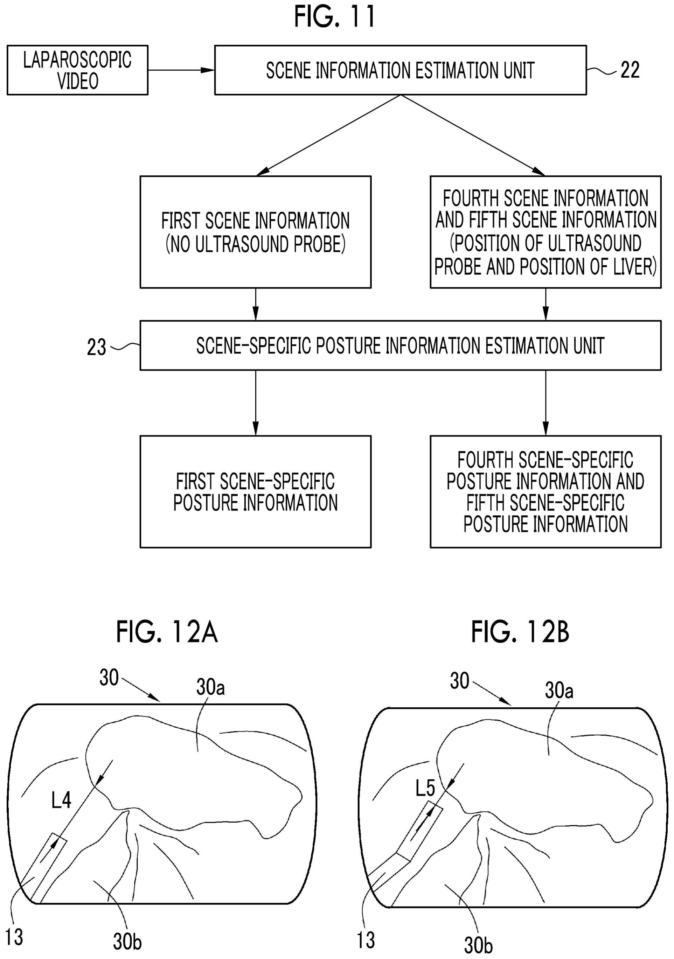

In addition, as shown in FIG. 11, in a case where the member is the ultrasound probe 13, the scene information estimation unit 22 estimates first scene information in a case where the ultrasound probe 13 is not included in the observation video, and the scene-specific posture information estimation unit 23 estimates first scene-specific posture information. On the other hand, in a case where the ultrasound probe 13 is included in the observation image, at least one of fourth scene information or fifth scene information is estimated according to the position of the ultrasound probe 13 and the position of the observation target organ. As shown in FIG. 12A, it is preferable that the fourth scene information be estimated in a case where an organ-probe distance L4 exceeds a second threshold value. In addition, as shown in FIG. 12B, it is preferable that the fifth scene information be estimated in a case where an organ-probe distance L5 is equal to or less than the second threshold value. It is preferable that the organ-probe distance be a distance between a distal end of the ultrasound probe 13 and the observation target organ (liver 30a).

The scene-specific posture information estimation unit 23 estimates at least one of fourth scene-specific posture information as the posture information based on the fourth scene information or fifth scene-specific posture information as the posture information based on the fifth scene information. In this case, the scene information estimation unit 22 may estimate a plurality of pieces of scene information without being limited to the two pieces of fourth scene information and fifth scene information. It is preferable that, in a case where the display controller 24 performs any of control of display based on the first scene-specific posture information, control of the display based on the fourth scene-specific posture information, or control of the display based on the fifth scene-specific posture information, any of first display, sixth display, or seventh display be performed as display of a 3D organ model.

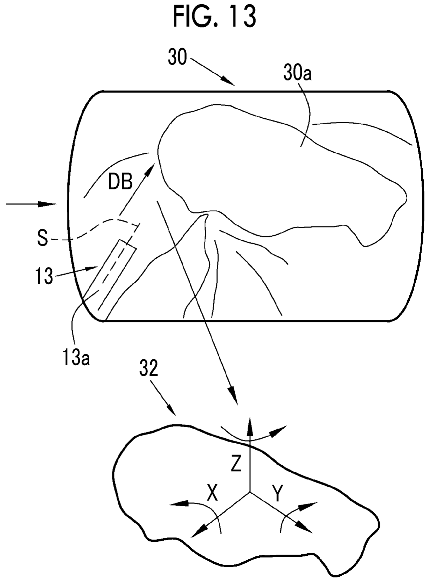

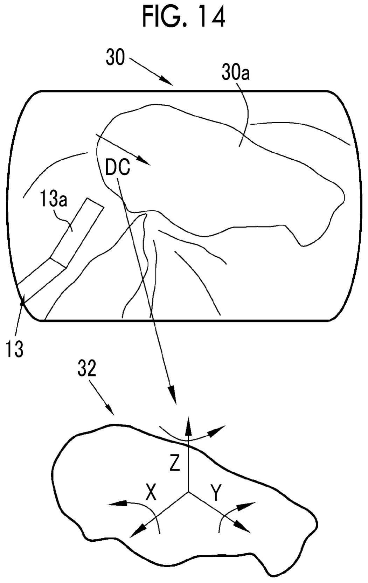

In the first display, as described above, a 3D organ model that changes in conjunction with the posture of the liver 30a is displayed (see FIG. 6). In the sixth display, as shown in FIG. 13, a 3D organ model as viewed from a direction DB orthogonal to (intersecting) a plane S (surface) orthogonal to (intersecting) an element surface 13a of the ultrasound probe 13 is displayed. It is preferable that the plane S be orthogonal to the element surface 13a and be parallel to a major axis direction of the element surface 13a. In the seventh display, as shown in FIG. 14, a 3D organ model as viewed from a direction DC orthogonal to (intersecting) the element surface 13a of the ultrasound probe 13 is displayed.

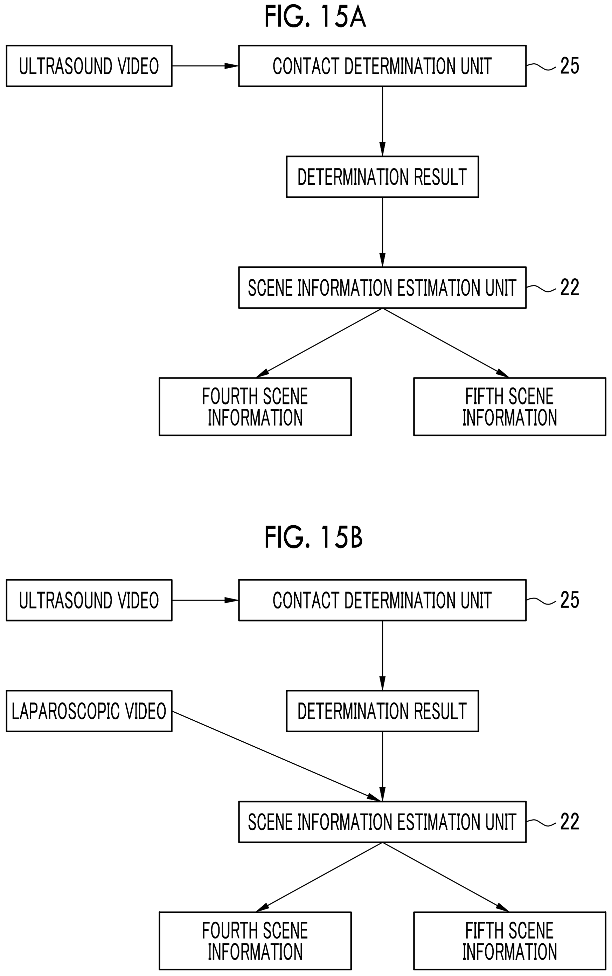

The scene information may be estimated by a method other than the organ-probe distance. For example, as shown in FIG. 15A, the contact determination unit 25 determines whether or not the ultrasound probe is in contact with the observation target organ based on the ultrasound video obtained from the ultrasound probe. The scene information estimation unit 22 estimates any of the fourth scene information or the fifth scene information according to the determination result. In this case, in a case where the ultrasound probe 13 is not in contact with the liver 30a, the fourth scene information is estimated, and, in a case where the ultrasound probe 13 is in contact with the liver 30a, the fifth scene information is estimated. As shown in FIG. 15B, the scene information estimation unit 22 may estimate any of the fourth scene information or the fifth scene information according to a combination of the determination result and the laparoscopic video.

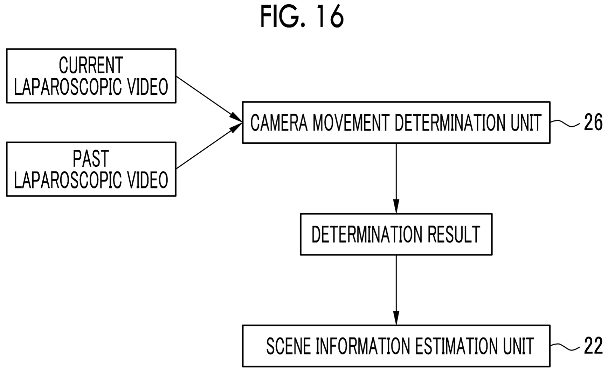

The camera movement determination unit 26 determines the movement of the camera from the laparoscopic video. In the present embodiment, the movement of the laparoscope 11 that serves as the camera is determined. The scene-specific posture information estimation unit 23 estimates scene-specific posture information in a case where it is determined that the camera has moved. In the present embodiment, as shown in FIG. 16, the camera movement determination unit 26 determines the movement of the laparoscope 11, which serves as the camera, from the current laparoscopic video and the past laparoscopic video. Then, the scene-specific posture information estimation unit 23 estimates scene-specific posture information in a case where it is determined that the laparoscope 11 has moved. For example, even in a case where the surgical tool-organ distance changes, in a case where it is determined that the laparoscope 11 has not moved, the scene-specific posture information that has already been estimated is used, and new scene-specific posture information is not estimated. This suppresses frequent switching of the posture of the 3D organ model in a case of changing the surgical tool 12 during a surgical treatment using the surgical tool 12.

The determination of the movement of the laparoscope 11 may be performed by detecting the current and past positions of the laparoscope 11 (or the positions of the laparoscope 11 estimated in the current and past) from the laparoscopic video, the sensor, or the like, and comparing the current and past positions of the laparoscope 11 (or the positions of the laparoscope 11 estimated in the current and past). In addition, the camera movement determination unit 26 may estimate the movement amount of the laparoscope 11 and determine the occurrence of the movement of the camera based on the estimated movement amount.

In the above-described embodiment, the scene information estimation unit 22 estimates the scene information, and the scene-specific posture information estimation unit 23 estimates the scene-specific posture information, but the method, the model, or the fixed value for estimating the posture information of the 3D organ model may be switched depending on the scene information. In this case, the display controller 24 controls the display of the 3D organ model in accordance with the switching.

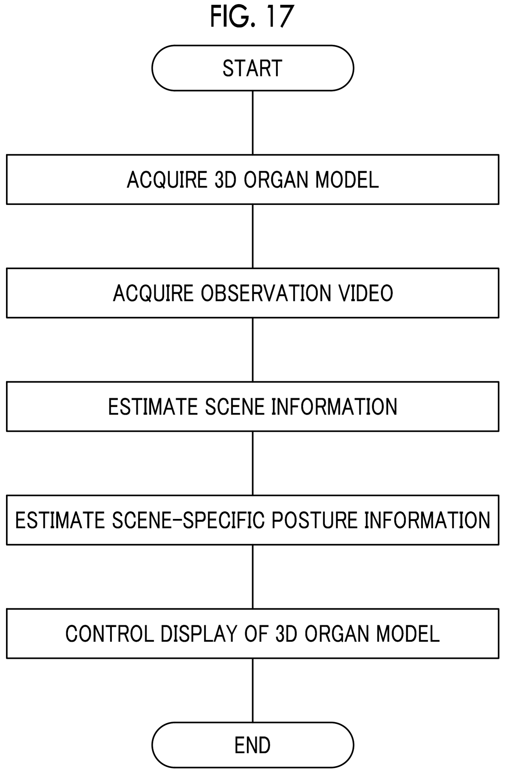

Next, a series of flows of the present disclosure will be described with reference to a flowchart of FIG. 17. The 3D organ model acquisition unit 20 acquires a 3D organ model corresponding to the observation target organ. The observation video acquisition unit 21 acquires an observation video of an observation target including the observation target organ. The scene information estimation unit 22 estimates scene information determined based on a position of the observation target organ or a positional relationship between the observation target organ and a member used for the observation target organ, from the laparoscopic video. The scene-specific posture information estimation unit 23 estimates posture information of the 3D organ model based on the scene information as scene-specific posture information. The display controller 24 controls the display of the 3D organ model on the display 16 based on the scene-specific posture information. The above-described series of flows is repeatedly performed until the observation is ended.

In the present embodiment, each process of the 3D organ model acquisition unit 20, the observation video acquisition unit 21, the scene information estimation unit 22, the scene-specific posture information estimation unit 23, the display controller 24, the contact determination unit 25, and the camera movement determination unit 26 is executed by any computer. In addition, any computer may execute the processing using a processor, a program, or a combination thereof. Any computer may be a general-purpose computer, a computer for a specific use, a system such as a workstation, or other hardware elements capable of executing a program.

The processor may be configured by one or more pieces of hardware, and the type of hardware is not limited. For example, the processor may be configured by a programmable logic device such as a central processing unit (CPU), a micro processing unit (MPU), or a field programmable gate array (FPGA), a dedicated circuit for executing specific processing such as an application specific integrated circuit (ASIC), or hardware such as a graphics processing unit (GPU) or a neural processing unit (NPU). In addition, the processor has each unit or each means that executes various types of processing in the present embodiment. In addition, the types of hardware may be a combination of different types of hardware. In a case where a plurality of pieces of hardware are configured to execute one or a plurality of processes of a certain processor, the plurality of pieces of hardware may be present in devices physically separated from each other, or may be present in the same device. In addition, in any of the embodiments, the order of each processing executed by the processor is not limited to the above order and may be changed as appropriate. The hardware is configured by an electric circuit (circuitry) in which circuit elements such as semiconductor elements are combined.

Further, the present embodiment may be realized by hardware, software, firmware, microcode, or a combination thereof. Software, firmware, and microcode are configured by a program. In addition, the program may be, for example, a program module group, and each function thereof may be realized by a processor configured to execute each function. The program may be a program code or a plurality of code segments stored in one or a plurality of non-transitory computer-readable media (for example, a recording medium or other storage). The program may be divided and stored in a plurality of non-transitory computer-readable media present in devices physically separated from each other. The program code or the code segment may represent any combination of a procedure, a function, a subprogram, a routine, a subroutine, a module, a software package, a class, an instruction, a data structure, or a program statement. The program code or the code segment may be connected to another code segment or a hardware circuit by transmitting and receiving information, data, an argument, a parameter, or memory contents.

### Explanation of References

10: medical system
11: laparoscope
12: surgical tool
13: ultrasound probe
13a: element surface
14: medical device
15: medical image processing device
16: display
17: user interface
20: 3D organ model acquisition unit
21: observation video acquisition unit
22: scene information estimation unit
23: scene-specific posture information estimation unit
24: display controller
25: contact determination unit
26: camera movement determination unit
30: laparoscopic video
30a: liver
30b: structure
30x: planned resection surface
30y: blood vessel of interest
32: 3D organ model
32a: liver
32b: blood vessel
NT: network
L2, L3: surgical tool-organ distance
L4, L5: organ-probe distance
VL1, VL2, VL3: line of sight
DA, DB, DC: direction
CA, CB: center
MT: movement
S: plane

## Claims

1. A medical device comprising:
a processor,
wherein the processor
acquires a 3D organ model corresponding to an observation target organ,
acquires an observation video of an observation target including the observation target organ,
estimates scene information determined based on a position of the observation target organ or a positional relationship between the observation target organ and a member used for the observation target organ, from the observation video,
estimates, as scene-specific posture information, posture information of the 3D organ model based on the scene information, and
controls display of the 3D organ model based on the scene-specific posture information.

2. The medical device according to claim 1,
wherein the member is a surgical tool used for resection of the observation target organ, and
the processor
estimates, in a case where the surgical tool is not included in the observation video, first scene information according to a relative posture between a camera that captures the observation video and the observation target organ, and estimates first scene-specific posture information as the posture information based on the first scene information, and
estimates, in a case where the surgical tool is included in the observation video, at least one of second scene information or third scene information according to either the positions of the surgical tool and the observation target organ or a surgical tool-organ distance between the surgical tool and the observation target organ, and estimates at least one of second scene-specific posture information as the posture information based on the second scene information or third scene-specific posture information as the posture information based on the third scene information.

3. The medical device according to claim 2,
wherein the second scene information is estimated in a case where the surgical tool-organ distance exceeds a first threshold value, and
the third scene information is estimated in a case where the surgical tool-organ distance is equal to or less than the first threshold value.

4. The medical device according to claim 2,
wherein, in a case where the processor performs control of the display based on any of the first scene-specific posture information, the second scene-specific posture information, or the third scene-specific posture information, any of the following displays is performed:
a first display, in which the 3D organ model that changes in conjunction with a posture of the observation target organ is displayed;
a second display, in which the 3D organ model as viewed from a line of sight connecting a distal end part of the surgical tool and a center of a planned resection surface of the observation target organ is displayed;
a third display, in which the 3D organ model as viewed from a direction of the planned resection surface of the observation target organ is displayed;
a fourth display, in which the 3D organ model as viewed from a line of sight connecting the distal end part of the surgical tool and a blood vessel of interest included in the observation target organ is displayed; or
a fifth display, in which the 3D organ model as viewed from a line of sight connecting the distal end part of the surgical tool and a center or center of gravity of the observation target organ is displayed.

5. The medical device according to claim 1,
wherein the member is an ultrasound probe, and
the processor
estimates, in a case where the ultrasound probe is not included in the observation video, first scene information according to a relative posture between a camera that captures the observation video and the observation target organ, and estimates first scene-specific posture information as the posture information based on the first scene information, and
estimates, in a case where the ultrasound probe is included in the observation video, at least one of fourth scene information or fifth scene information according to either the positions of the ultrasound probe and the observation target organ or an organ-probe distance between the ultrasound probe and the observation target organ, and estimates at least one of fourth scene-specific posture information as the posture information based on the fourth scene information or fifth scene-specific posture information as the posture information based on the fifth scene information.

6. The medical device according to claim 5,
wherein the fourth scene information is estimated in a case where the organ-probe distance exceeds a second threshold value, and
the fifth scene information is estimated in a case where the organ-probe distance is equal to or less than the second threshold value.

7. The medical device according to claim 5,
wherein, in a case where the processor performs control of the display based on any of the first scene-specific posture information, the fourth scene-specific posture information, or the fifth scene-specific posture information, any of the following displays is performed:
a first display, in which the 3D organ model that changes in conjunction with a posture of the observation target organ is displayed;
a sixth display, in which the 3D organ model as viewed from a direction intersecting a surface intersecting an element surface of the ultrasound probe is displayed; or
a seventh display, in which the 3D organ model as viewed from a direction intersecting the element surface of the ultrasound probe is displayed.

8. The medical device according to claim 5,
wherein the processor
determines whether or not the ultrasound probe is in contact with the observation target organ based on an ultrasound video obtained from the ultrasound probe, and
estimates either the fourth scene information or the fifth scene information according to either the result of the determination or a combination of the result of the determination and the observation video.

9. The medical device according to claim 1, further comprising:
a camera that captures the observation video,
wherein the processor
determines movement of the camera, and
estimates the scene-specific posture information in a case where it is determined that the camera has moved.

10. An operation method of a medical device including a processor, the operation method comprising causing the processor to execute:
a step of acquiring a 3D organ model corresponding to an observation target organ;
a step of acquiring an observation video of an observation target including the observation target organ;
a step of estimating scene information determined based on a position of the observation target organ or a positional relationship between the observation target organ and a member, from the observation video;
a step of estimating, as scene-specific posture information, posture information of the 3D organ model based on the scene information; and
a step of controlling display of the 3D organ model based on the scene-specific posture information.
